# EUROPEAN PATENT SPECIFICATION

(11) **EP 0 379 195 B1**
(45) Date of publication and mention of the grant of the patent: **10.08.1994**
(21) Application number: 90101014.0
(22) Date of filing: 18.01.1990
(51) Int. Cl.: A61B 6/00

(54) **X-ray fluoroscopy apparatus**
Röntgengerät zur Fluoroskopie
Appareil à rayons X pour fluoroscopie

(30) Priority: 20.01.1989 JP 11627/89
(43) Date of publication of application: 25.07.1990
(73) Proprietor: KABUSHIKI KAISHA TOSHIBA, Kawasaki-shi, Kanagawa-ken 210 (JP)
(72) Inventor: Takahata, Hideo, c/o Intellectual Property Div., Minato-ku, Tokyo 105 (JP)
(74) Representative: Blumbach, Kramer & Partner

(56) References cited:
- EP-A- 0 135 723
- EP-A- 0 146 991
- EP-A- 0 178 661
- EP-A- 0 287 117
- FR-A- 1 335 544
- FR-A- 2 075 430
- US-A- 4 365 345

## Description

The present invention relates to an X-ray fluoroscopy apparatus comprising:
a table on which a patient can be laid and which extends along a line;
an X-ray source for shooting X-ray to the patient;
a spot device arranged to face the X-ray source with the patient interposed between them and having a sheet of film on which X-ray penetrated through intended parts of the patient is projected, so that images of these intended parts of the patient are formed thereon; and
means for moving the X-ray source and the spot device along the table extending line and stopping them at plural predetermined positions along the table extending line comprising electrical motors;
means for controlling the X-ray source, the spot device, and the moving means to repeat the operation of moving the X-ray source and the spot device to one of their stop positions, shooting X-ray to one of the intended parts of the patient to form the image of this part on the film while keeping the X-ray source and the spot device stopped at this stop position for a predetermined time period, and then moving the X-ray source and the spot device to the next stop position,
and relates to a method for controlling an X-ray fluoroscopy apparatus.

In the case of the common X-ray fluoroscopy apparatus, a patient is laid on a table top which can move on the bed. A relatively small amount of X-ray is shot to the patient through an X-ray source and the X-ray which has penetrated through the patient is converted to optical signal by an image intensifier. The image of the patient penetrated with X-ray is displayed on a television monitor in response to the optical signal. The operator determines that part of the patient which is to be photographed with X-ray, viewing the television monitor, and the table top is moved to hold this part of the patient facing the X-ray source. A relatively large amount of X-ray is shot to the part of the patient from the X-ray source to photograph the X-ray image of the part on a sheet of film in a spot device.

The common X-ray fluoroscopy apparatus is usually used to photograph the system of digestive organ of the patient. However, it is sometimes used to photograph the circulatory system, more specifically, to angiograph the lower limb of the patient (to successively photograph images of blood vessels in all areas of the lower limb of the patient). Image forming agent is injected into a blood vessel and it flows through blood vessels in the lower limb of the patient at a relatively high speed. The patient is moved following the flow of image forming agent in the blood vessels and those blood vessels through which image forming agent is flowing are successively photographed with X-ray.

For the purpose of meeting this object, the common X-ray fluoroscopy apparatus is provided with another X-ray source and a film changer located beside its bed. When image forming agent is to be injected into a certain part (or the thigh, for example) of the patient through a catheter, this certain part of the patient is displayed on a screen of the television camera through an image intensifier. The position of injecting image forming agent into the part of the patient is thus determined, viewing the X-ray fluoroscopic image of the part of the patient on the television camera. The catheter is inserted into the patient at this position and image forming agent is injected into blood vessels in the thigh of the patient through the catheter. Just after the injection of image forming agent into the patient, the table top is moved to hold the catheter-stuck part of the patient facing the another X-ray source. X-ray is shot to the part of the patient from the another X-ray source to photograph blood vessels in the patient's thigh, through which image forming agent is flowing, on the sheet of film in the film changer.

It is needed to successively photograph images of blood vessels, following the flow of image forming agent in these blood vessels. The table top is thus further moved by a certain distance to hold the other part of those blood vessels, through which image forming agent is flowing, facing the another X-ray source. At the same time, the sheet of film on which an X-ray image of blood vessels has been photographed is exchanged with a new one in the film changer. X-ray is shot under this state to the other part of blood vessels to photograph it on the new sheet of film. The moving of the table top, exchanging of the film with a new one and shooting of X-ray are repeated three or four times in this manner. Images of those blood vessels in the patient through which image forming agent is flowing can be thus photographed, following the flow of image forming agent in the blood vessels of the patient.

When the lower limb of the patient is to be angiographed by the common X-ray fluoroscopy apparatus, however, another X-ray source and a film changer are needed, as described above. This causes the apparatus to be made complicated and cost high.

Further, the table top must be moved following the speed of image forming agent flowing through blood vessels. More specifically, the table top must be moved at a relatively high speed which is about three times the speed of the table top moved when the patient on it is under normal photographing process. This causes the patient to feel remarkable pain.

In the FR-A-2 075 430 an X-ray fluoroscopy apparatus is disclosed being similar to the apparatus according to the preamble of claim 1 and in which the X-ray source and the film changer are moved automatically in order to take images of intended parts of the patient. However, it is not disclosed how to control the X-ray source and the film changer during automatic motion.

From prior art, US-A-4,365,345 it is known to employ a potentiometer assembly to control a constant alignment of an X-ray generator and an X-ray receptor during motion. Both components are comprising a potentiometer, respectively, whereby the potentiometers are part of a bridging circuit controlling a servo-amplifier in order to move both components properly aligned to each other. The speed of the X-ray fluoroscopy apparatus is not controlled by the potentiometers, only an undesired difference in the position between moving parts is avoided.
According to the present invention, there can be provided an X-ray flouroscopy apparatus as disclosed in the preamble of claim 1, in which said moving means includes a servo-motor for outputting intermittent drive force, and means for transmitting the intermittent drive force from the servo-motor to the X-ray source and the spot device to intermittently move the X-ray source and the spot device, whereby the moving speed of the X-ray source and the spot device is controlled by means of a tachometer generator controlling the speed of said servo-motor.

When the circulatory system is photographed, more specifically, the lower limb of the patient is angiographed, the X-ray source and the spot device are moved to one of their stop positions at a speed suitable for photographing blood vessels in the lower limb of the patient, and X-ray is shot to an intended part of the lower limb to photograph blood vessels in the intended part of the patient on a film, while keeping the X-ray source and the spot device stopped at this stop position for a predetermined time period, and then moving them to their next stop position. The process is repeated to photograph those blood vessels through which image forming agent is flowing every time when the X-ray source and the spot device are moved from one of their stop positions to the others. Images of blood vessels through which image forming agent is flowing can be thus photographed, following the flow of image forming agent in the blood vessels.

According to the present invention, therefore, neither additional X-ray source nor film changer is needed. This enables the apparatus to be made simpler and cost lower. Further, it is not needed to move the patient on the table top and this prevents the patient from feeling pain as often seen in the conventional case. This also makes it unnecessary to move the table top together with the patient, thereby enabling the table top to be made relatively shorter.

This invention can be more fully understood from the following detailed description when taken in conjunction with the accompanying drawings, in which:
Fig. 1 shows an example of the X-ray fluoroscopy apparatus according to the present invention;
Fig. 2 is a perspective view showing a device for moving an X-ray source and a spot device shown in Fig. 1;
Fig. 3 is a block diagram showing a device for controlling the moving device shown in Fig. 2;
Fig. 4 is a flow chart for the control device shown in Fig. 3; and
Fig. 5 is a time chart for the amount of image forming agent injected and the X-ray shot.

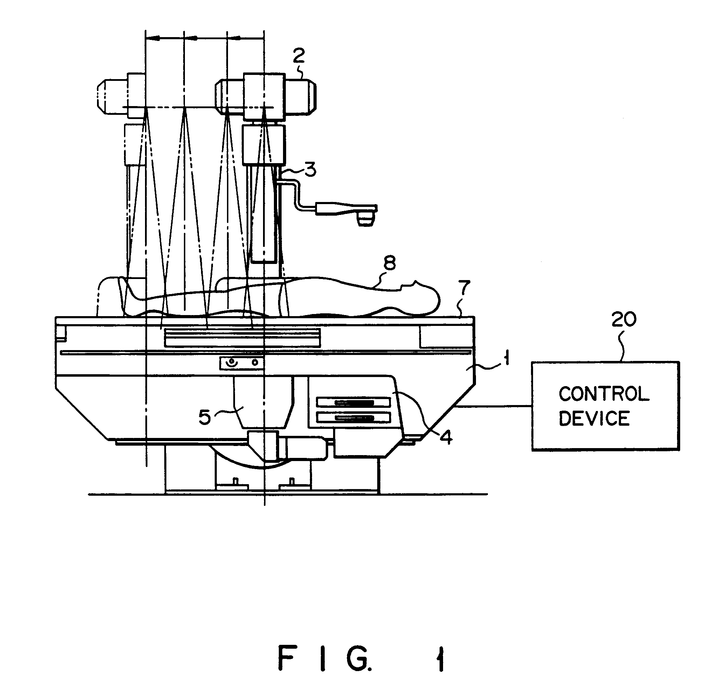

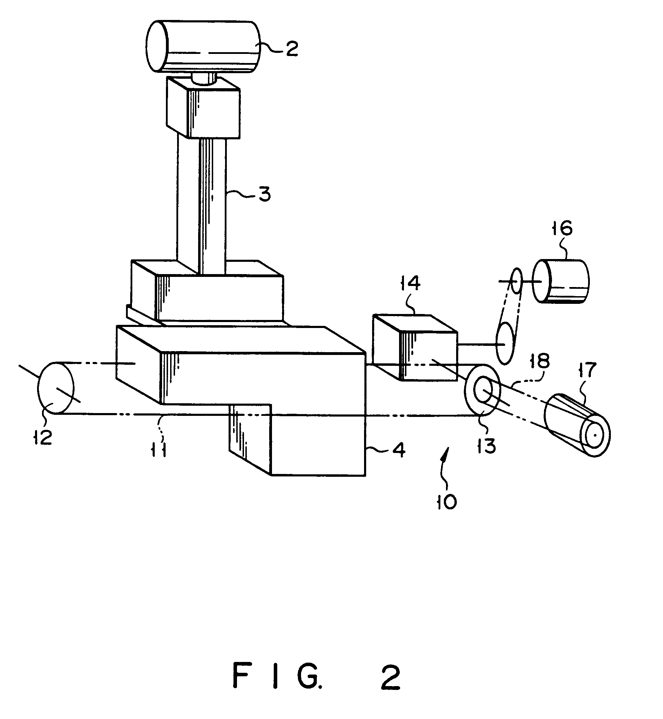

Fig. 1 shows an example of the X-ray fluoroscopy apparatus according to the present invention. This photographing apparatus includes body 1 provided with table top 7 movable to carry patient 8 thereon. Body 1 has support 3, to the top of which is attached X-ray tube (or source) 2 for shooting X-ray to patient 8. Spot device 4 is attached to the lower end of support 3, as shown in Fig. 2. Spot device 4 is located to face X-ray tube 2 with patient 8 interposed between them, serving to receive X-ray, which has penetrated patient 8, on a film and transfer images of patient 8 onto it. The film in spot device 4 is divided into plural (three or four) sections. When patient 8 is to be photographed with X-ray, each of these film sections is successively moved to a position where the X-ray which has penetrated through patient 8 is projected on it, so that images of patient 8 thus photographed with X-ray can be fixed on their respective sections of the film. Support 3 can move X-ray tube 2 up and down to change that area of patient 8 which is photographed with X-ray. Body 1 further includes image intensifier 5 for converting the X-ray, which has penetrated through patient 8, to optical signals and displaying penetrated images of patient 8, which are formed responsive to these optical signals, on television camera.

Fig. 2 shows system 10 for moving X-ray tube 2 and spot device 4 in a direction parallel to table top 7. This system 10 is housed in body 1. System 10 includes servo-motor 16 which can output intermittent drive force at high speed. A chain is stretched between the driving shaft of servo-motor 16 and the input shaft of speed reduction means 14. Driving sprocket 13 is attached to the output shaft of speed reduction means 14. Chain 11 is stretched between driving sprocket 13 and idler sprocket 12 arranged in body 1. Spot device 4 is attached to this chain 11.

When servo-motor 16 outputs intermittent drive force, this intermittent drive force is transmitted to sprocket 13 through speed reduction means 14 and the liken to intermittently move chain 11. X-ray tube 2 and spot device 4 can be thus intermittently moved in the direction parallel to table top 7, at the lower limb angiography.

Chain 18 is stretched between sprocket 13 and encoder 17. Positions of X-ray tube 2 and spot device 4 can be detected by this encoder 17.

According to the present invention, at the lower limb angiography, X-ray tube 2 and spot device 4 are intermittently moved, and X-ray is shot to patient 8 every time when X-ray tube 2 and spot device 4 are stopped at four positions, as shown in Fig. 1. The following items must be therefore taken into consideration to control the X-ray photographing apparatus.
1) X-ray tube 2 and spot device 4 are stopped at their initial position when photographing is to be started.
2) X-ray tube 2 and spot device 4 are moved by a predetermined distance when they are to be moved from a position to a next position.
3) X-ray tube 2 and spot device 4 are moved at a predetermined speed when they are to be moved.
4) X-ray tube 2 and spot device 4 are stopped at their respective positions for a predetermined time period.
5) The timing of X-ray shot is controlled while X-ray tube 2 and spot device 4 are stopped at their respective positions.
6) Each of sections of the film is set at that position which can receive the X-ray penetrated through an intended part of patient 8, while spot device 4 is being moved from a position to a next position.

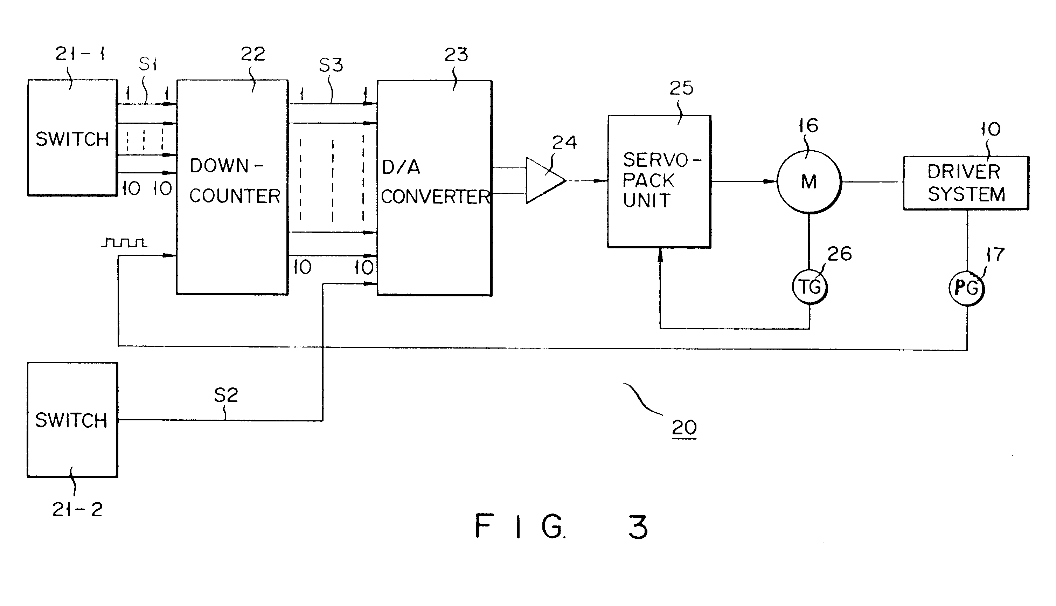

For the purpose of satisfying these items, the X-ray photographing apparatus according to the present invention is provided with device 20 for controlling system 10, as shown in Fig. 3. Another device for controlling X-ray tube 2 and spot device 4 is not shown for the sake of clarification.

Control device 20 is provided with switch 21-1 to which the moving distance of X-ray tube 2 and spot device 4 is inputted. Switch 21-1 outputs signal S₁ which consists of ten bits to set the moving distance of X-ray tube 2 and spot device 4.

Control device 20 is further provided with switch 21-2 for causing the X-ray photographing apparatus to start its photographing blood vessels of the lower limb of patient 8. When switch 21-2 is switched on, signal S₂ for causing X-ray tube 2 and spot device 4 to be intermittently moved at high speed is supplied from switch 21-2 to D/A converter 23.

Signal S₁ is applied to down-counter 22 and encoder signal is also applied from encoder 17 to this down-counter 22. Down-counter 22 counts down the value of distance by which X-ray tube 2 and spot device 4 are moved to their initial position, and it outputs count signal S₃. When X-ray tube 2 and spot device 4 are not positioned at their initial position, therefore, difference between their initial position and their now-stopped position is detected. As the result, information of this difference is supplied to servo-pack unit 25, causing X-ray tube 2 and spot device 4 to be moved to their initial position.

D/A converter 23 converts signals S₃ and S₂ applied from down-counter 22 and switch 21-2, respectively, to analog signals. Amplifier 24 amplifies these analog signals applied from D/A converter 23 to a predetermined level. Signal is applied from amplifier 24 to servo-pack unit 25 while speed information of servo-motor 16 is also applied from tachometer generator 26 to servo-pack unit 25, thereby enabling the intermittent run and speed of servo-motor 16 to be controlled. X-ray tube 2 and spot device 4 can be therefore intermittently moved and stopped at their four positions, as shown in Fig. 1.

The moving speed and stopping time period of X-ray tube 2 and spot device 4 are previously set. Their moving speed corresponds to the speed of image forming agent flowing through blood vessels of the lower limb of patient 8.

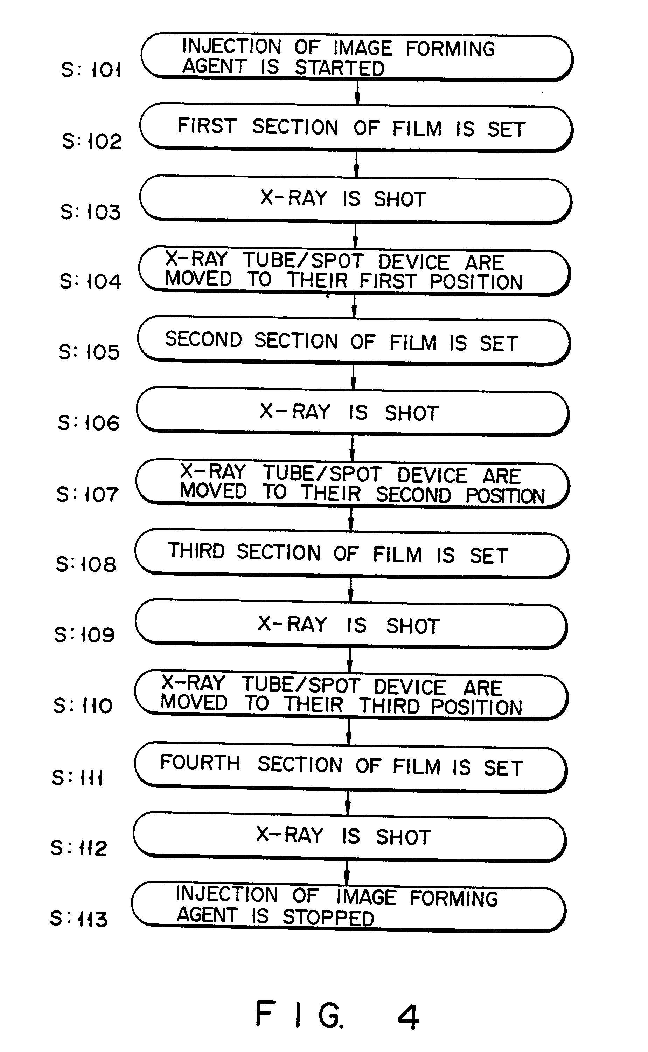

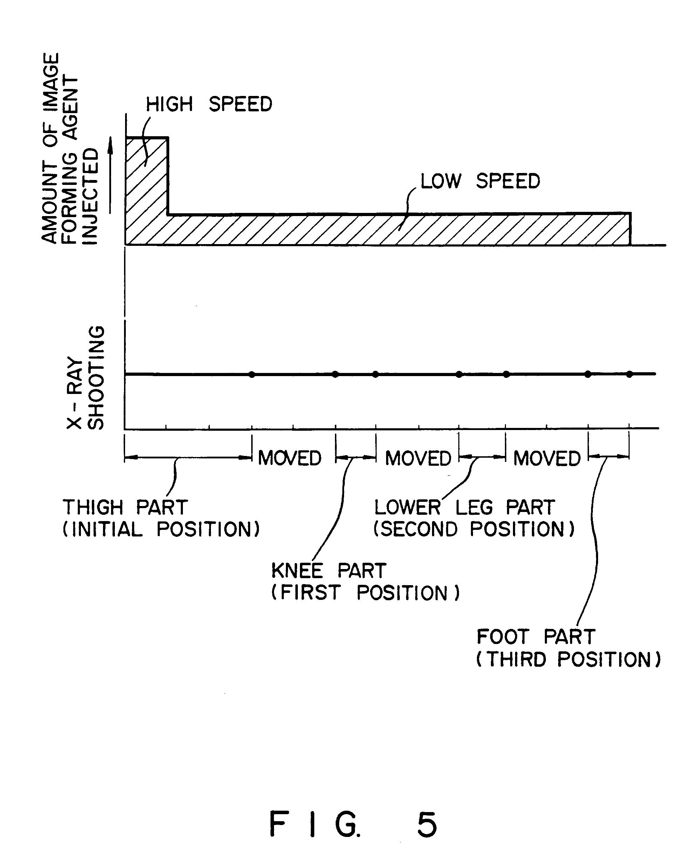

It will be described how blood vessels of the lower limb of patient 8 are photographed by the X-ray penetration photographing apparatus of the present invention, referring to the flow chart shown in Fig. 4 and the time chart shown in Fig. 5.

Moving distances of X-ray tube 2 and spot device 4 are inputted to switch 21-1 by the operator. Information of these moving distances is thus inputted to servo-pack unit 25 through down-counter 22 and the like. When X-ray tube 2 and spot device 4 are not positioned at their initial position, they are moved to their initial position.

A predetermined part (or thigh) of patient 8 is displayed on TV camera through image intensifier 5. A catheter is put into the predetermined part of patient 8 and image forming agent is injected into a blood vessel in the thigh of patient 8 through the catheter at step 101, while viewing TV camera. A relatively large amount of image forming agent is injected this time into the blood vessel at high speed, as shown in Fig. 5. The image forming agent whose amount has been reduced to a relatively small level is then injected into the blood vessel at low speed.

Switch 21-2 is switched on at step 102. The first section of the film is thus set at the predetermined position in spot device 4. X-ray is shot to the part of patient 8 through X-ray tube 2 at step 103. The image of those blood vessels through which the image forming agent is flowing is thus photographed on the first section of the film.

X-ray tube 2 and spot device 4 are moved to their first stop position (or the knee part of patient 8) at step 104. The second section of the film is set this time at its predetermined position in spot device 4. When X-ray tube 2 and spot device 4 reaches their first position and they are kept stopped there (or at the knee part of patient 8), X-ray is shot to the knee part of patient 8 through X-ray tube 2. X-ray may be shot twice, as shown in Fig. 5.

This process is repeated at steps 107 through 112. More specifically, X-ray tube 2 and spot device 4 are moved to their second and then third positions (or the lower leg and then foot parts of patient 8) while positioning the third and then fourth sections of the film at the predetermined position in spot device 4. When they are at their second and third positions X-ray is shot through X-ray tube 2 to photograph images of blood vessels in the lower leg and foot parts of patient 8, through which the image forming agent is flowing, on the third and fourth sections of the film, respectively. The injection of image forming agent is stopped at step 113.

The intermittent moving of X-ray tube 2 and spot device 4, setting of the film and shooting of X-ray are repeated in this manner, so that those blood vessels in the respective parts of patient 8 through which the image forming agent is flowing can be photographed on the respective sections of the film every time when X-ray tube 2 and spot device 4 are moved.

According to the present invention which is intended to angiograph the lower limb of patient 8 through X-ray tube 2 in this example, X-ray tube 2 and spot device 4 are moved to each of their positions at speed suitable for photographing the lower limb of patient 8. In addition, X-ray is shot to the intended respective parts of patient 8 to photograph images of blood vessels in these parts on the respective sections of the film, while keeping X-ray tube 2 and spot device 4 stopped at their respective positions for the predetermined time period. Neither the X-ray tube nor a film changer is therefore needed to be provided independently of the X-ray photographing apparatus. This enables the apparatus to be made simpler and cost lower. Further, it is not needed to move patient 8 on table top 7. This prevents patient 8 from feeling pain and enables table top 7 to be made shorter.

Furthermore, it is not needed to move the thigh of patient 8 to the position of the film changer after image forming agent is injected into the thigh of patient 8, while viewing the image of the thigh displayed on the screen of TV camera through image intensifier 5. This enables images of blood vessels to be photographed just after image forming agent is injected into them.

It should be understood that the present invention is not limited to the above-described embodiment. Particularly, those positions at which X-ray tube 2 and spot device 4 are stopped are not limited to three or four. They may be more or less.

## Claims

1. An X-ray fluoroscopy apparatus comprising:
a table (7) on which a patient (8) can be laid and which extends along a line;
an X-ray source (2) for shooting X-ray to the patient (8);
a spot device (4) arranged to face the X-ray source (2) with the patient interposed between them and having a sheet of film on which X-ray penetrated through intended parts of the patient is projected, so that images of these intended parts of the patient (8) are formed thereon; and
means (10) for moving the X-ray source (2) and the spot device (4) along the table extending line and stopping them at plural predetermined positions along the table extending line comprising electrical motors;
means (20) for controlling the X-ray source, the spot device, and the moving means to repeat the operation of moving the X-ray source (2) and the spot device (4) to one of their stop positions, shooting X-ray to one of the intended parts of the patient (8) to form the image of this part on the film while keeping the X-ray source (2) and the spot device (4) stopped at this stop position for a predetermined time period, and then moving the X-ray source (2) and the spot device (4) to the next stop position,
characterized in that said moving means (10) includes a servo-motor (16) for outputting intermittent drive force, and means for transmitting the intermittent drive force from the servo-motor (16) to the X-ray source (2) and the spot device (4) to intermittently move the X-ray source (2) and the spot device (4), whereby the moving speed of the X-ray source (2) and the spot device (4) is controlled by means of a tachometer generator (26) controlling the speed of said servo-motor (16).

2. The X-ray flouroscopy apparatus according to claim 1, characterized by further comprising an image intensifier (5) for receiving X-ray shot from the X-ray source (2) and penetrated through the intended part of the patient (8), converting it to optical signals, and displaying the image of this part, which has been penetrated with X-ray, on a screen of a television camera in response to the optical signals.

3. The X-ray fluoroscopy apparatus according to claim 1 or 2, characterized in that the film is divided into plural sections and the spot device (4) sets one of these sections of the film at a position where the X-ray is projected through one of the intended parts of the patient, before the X-ray source (2) and the spot device (4) are moved to one of their stop positions and X-ray is shot to the intended part of the patient (8).

4. The X-ray fluoroscopy apparatus according to claim 1, 2 or 3, characterized in that said control means (20) includes a servo-pack unit (25) for supplying control signals to the servo-motor (16) and a means for inputting information of moving distance and speed of the X-ray source (2) and the spot device (4) to the servo-pack unit (25) when the X-ray source (2) and the spot device (4) are to be moved from one of their spot positions to their next stop position.

5. The X-ray fluoroscopy apparatus according to one of claims 1 to 4, characterized in that the moving speed of the X-ray source (2) and the spot device (4) corresponds to the speed of image forming agent flowing through blood vessels in the intended parts of the patient (8).

6. The X-ray fluoroscopy apparatus according to claim 4, characterized in that said control means (20) includes a means for inputting signals to the servo-pack unit (25) to start its control; means for detecting the position of the X-ray source (2) and the spot device (4) stopped before the servo-pack unit (25) starts its control; and means for detecting difference between the initial position of the X-ray source (2) and the spot device (4), at which they begin to be controlled by the servo-pack unit (25), and their now-stopped position in response to information of their position now stopped to supply information of this difference to the servo-pack unit (25) to move them to their initial position.

7. The X-ray fluoroscopy apparatus according to claim 6, characterized in that said control means (20) causes X-ray to be shot to one of the intended parts of the patient (8) to photograph the intended part of the patient (8) on one of the plural sections of the film before it starts to move the X-ray source (2) and the spot device (4) to their first stop position.

8. A method for controlling an X-ray fluoroscopy apparatus according to claim 1, said method comprising steps of:
moving the X-ray source (2) and the spot device (4) to one of their stop positions;
shooting the X-ray to one of the intended parts of the patient (8) to photograph this part on the film while keeping the X-ray source (2) and the spot device (4) stopped at this stop position for a predetermined time period; and
moving the X-ray source (2) and the spot device (4) to their next stop position, characterized by controlling the moving speed of the X-ray source (2) and the spot device (4) by means of a tachometer generator (26) controlling the speed of said servo-motor (16).

## Patentansprüche

1. Röntgenbildschirmgerät, umfassend:
einen Tisch (7), auf den ein Patient (8) gelegt werden kann und der sich längs einer Linie erstreckt;
eine Röntgenstrahlungsquelle (2) zum Abgeben von Röntgenstrahlung an den Patienten (8);
eine Bildpunkteinrichtung (4), die mit dazwischen angeordnetem Patienten Röntgenstrahlungsquelle (2) gegenüber liegt, und die einen Filmbogen hat, auf den Röntgenstrahlung, die durch die zu untersuchenden Körperteile des Patienten hindurchgetreten ist, projiziert wird, so daß Bilder dieser zu untersuchenden Körperteile des Patienten (8) darauf abgebildet werden; und
eine Einrichtung (10) zum Bewegen der Röntgenstrahlungsquelle (2) und der Bildpunkteinrichtung (4) längs der Linie, entlang der sich der Tisch erstreckt, und zum Anhalten derselben an einer Anzahl vorbestimmter Stellen längs der Linie, entlang der sich der Tisch erstreckt, umfassend elektrische Motoren;
eine Einrichtung (20) zum Steuern der Röntgenstrahlungsquelle, der Bildpunkteinrichtung und der Einrichtung zum Bewegen, um den Vorgang des Bewegens der Röntgenstrahlungsquelle (2) und der Bildpunkteinrichtung (4) zu einer ihrer Haltestellen zu wiederholen, wobei Röntgenstrahlung an einen der zu untersuchenden Körperteile des Patienten (8) abgegeben wird, um das Bild von diesem Körperteil auf den Film abzubilden, während die Röntgenstrahlungsquelle (2) und die Bildpunkteinrichtung (4) an dieser Haltestelle für eine vorbestimmte Zeitdauer angehalten werden, und dann die Röntgenstrahlungsquelle (2) und die Bildpunkteinrichtung (4) zu ihrer nächsten Haltestelle bewegt werden, dadurch **gekennzeichnet**, daß
die Einrichtung (10) zum Bewegen einen Servo-Motor (16) zum Abgeben einer intermittierenden Antriebskraft und eine Einrichtung zum Übertragen der intermittierenden Antriebskraft von dem Servo-Motor (16) an die Röntgenstrahlungsquelle (2) und die Bildpunkteinrichtung (4) umfaßt, um die Röntgenstrahlungsquelle (2) und die Bildpunkteinrichtung (4) intermittierend zu bewegen, wobei die Bewegungsgeschwindigkeit der Röntgenstrahlungsquelle (2) und der Bildpunkteinrichtung (4) mittels eines Tachometergenerators (26) überwacht wird, um die Geschwindigkeit des Servo-Motors (16) zu steuern.

2. Röntgenbildschirmgerät gemäß Anspruch 1, dadurch **gekennzeichnet**, daß
es ferner umfaßt
einen Bildverstärker (5) zum Aufnehmen von Röntgenstrahlung, die von der Röntgenstrahlungsquelle (2) abgegeben worden und durch das beabsichtigte Körperteil des Patienten (8) hindurchgetreten ist, zum Umwandeln der Röntgenstrahlung in optische Signale und zum Darstellen des Bildes von diesem Körperteil, durch den die Röntgenstrahlung hindurchgetreten ist, auf einem Bildschirm einer Fernsehkamera nach Maßgabe der optischen Signale.

3. Röntgenbildschirmgerät nach Anspruch 1 oder 2, dadurch **gekennzeichnet**, daß
der Film in mehrere Abschnitte unterteilt ist, und die Bildpunkteinrichtung (4) einen dieser Abschnitte des Films an eine Position setzt, wo die Röntgenstrahlung durch einen der zu untersuchenden Körperteile des Patienten projiziert wird, bevor die Röntgenstrahlungsquelle (2) und die Bildpunkteinrichtung (4) zu einer ihrer Haltepositionen bewegt werden, und Röntgenstrahlung zu dem zu untersuchenden Körperteile des Patienten (8) abgegeben wird.

4. Röntgenbildschirmgerät nach Anspruch 1, 2 oder 3, dadurch **gekennzeichnet**, daß
die Steuereinrichtung (20) eine Servo-Pack-Einheit (25) umfaßt zum Abgeben von Steuersignalen an den Servo-Motor (16) und eine Einrichtung zum Eingeben von Informationen über die Bewegungsstrecke und die Bewegungsgeschwindigkeit der Röntgenstrahlungsquelle (2) und der Bildpunkteinrichtung (4) an die Servo-Pack-Einheit (25), wenn die Röntgenstrahlungsquelle (2) und die Bildpunkteinrichtung (4) von einer ihrer Punktpositionen zu ihrer nächsten Punktposition bewegt werden.

5. Röntgenbildschirmgerät nach einem der Ansprüche 1 bis 4, dadurch **gekennzeichnet**, daß
die Bewegungsgeschwindigkeit der Röntgenstrahlungsquelle (2) und der Bildpunkteinrichtung (4) der Geschwindigkeit des bilderzeugenden Mittels entsprechen, das durch die Blutgefäße in den beabsichtigten Körperteilen des Patienten (8) fließt.

6. Röntgenbildschirmgerät nach Anspruch 4, dadurch **gekennzeichnet**, daß
die Steuereinrichtung (20) umfaßt
eine Einrichtung zum Eingeben von Signalen an die Servo-Pack-Einheit (25) zum Starten ihrer Steuerung;
eine Einrichtung zum Erfassen der Stelle, an der die Röntgenstrahlungsquelle (2) und die Bildpunkteinrichtung (4) angehalten worden sind, bevor die Servo-Pack-Einheit (25) ihre Steuerung beginnt; und
eine Einrichtung zum Erfassen des Unterschiedes zwischen der Anfangsposition der Röntgenstrahlungsquelle (2) und der Bildpunkteinrichtung (4), an der sie beginnen, durch die Servo-Pack-Einheit (25) gesteuert zu werden, und ihre, nun gestoppte Position nach Maßgabe der Information über ihre nun gestoppte Position, um die Information über diesen Unterschied an die Servo-Pack-Einheit (25) zuzuführen und diese an ihre Anfangsposition zu bewegen.

7. Röntgenbildschirmgerät nach Anspruch 6, dadurch **gekennzeichnet**, daß
die Steuereinrichtung (20) veranlaßt, daß Röntgenstrahlung an einen der zu untersuchenden Körperteile des Patienten (8) abgegeben wird, um diesen Körperteil des Patienten (8) auf einen der mehreren Abschnitte des Films zu fotografieren, bevor sie beginnt, die Röntgenstrahlungsquelle (2) und die Bildpunkteinrichtung (4) zu ihrer ersten Haltestelle zu bewegen.

8. Verfahren zum Steuern eines Röntgenbildschirmgeräts nach Anspruch 1, wobei das Verfahren folgende Schritte umfaßt:
Bewegen der Röntgenstrahlungsquelle (2) und der Bildpunkteinrichtung (4) zu einer ihrer Haltepositionen;
Abgeben der Röntgenstrahlung an einen der zu untersuchenden Körperteile des Patienten (8), um diesen auf dem Film zu fotografieren, während die Röntgenstrahlungsquelle (2) und die Bildpunkteinrichtung (4) an dieser Halteposition für eine vorbestimmte Zeitdauer angehalten werden; und
Bewegen der Röntgenstrahlungsquelle (2) und der Bildpunkteinrichtung (4) zu ihrer nächsten Halteposition, gekennzeichnet durch Überwachen der Bewegungsgeschwindigkeit der Röntgenstrahlungsquelle (2) und der Bildpunkteinrichtung (4) mittels eines Tachometergenerators (26) zum Steuern der Geschwindigkeit des Servo-Motors (16).

## Revendications

1. Un appareil de radiographie à rayons X comprenant :
une table (7) sur laquelle un patient (8) peut être étendu et qui s'étend le long d'une ligne;
une source de rayons X (2) pour projeter des rayons X sur le patient (8);
un dispositif de positionnement de film (4) qui est disposé face à la source de rayons X (2) avec le patient interposé entre eux, et qui porte une feuille de film sur laquelle des rayons X qui ont traversé des parties désirées du patient sont projetés de façon à former sur le film des images de ces parties désirées du patient (8); et
des moyens (10) pour déplacer la source de rayons X (2) et le dispositif de positionnement de film (4) le long de la ligne d'extension de la table et pour les arrêter à plusieurs positions prédéterminées le long de la ligne d'extension de la table, ces moyens comprenant des moteurs électriques;
des moyens (20) pour commander la source de rayons X, le dispositif de positionnement de film et les moyens de déplacement de façon à répéter l'opération qui consiste à déplacer la source de rayons X (2) et le dispositif de positionnement de film (4) jusqu'à l'une de leurs positions d'arrêt, à projeter des rayons X sur l'une des parties désirées du patient (8), pour former l'image de cette partie sur le film, tout en maintenant la source de rayons X (2) et le dispositif de positionnement de film (4) arrêtés à cette position d'arrêt pendant une durée prédéterminée, et à déplacer ensuite la source de rayons X (2) et le dispositif de positionnement de film (4) jusqu'à la position d'arrêt suivante,
caractérisé en ce que les moyens de déplacement (10) comprennent un servomoteur (16) qui est destiné à produire une force d'entraînement intermittente, et des moyens pour transmettre la force d'entraînement intermittente du servomoteur (16) à la source de rayons X (2) et au dispositif de positionnement de film (4), pour déplacer par intermittence la source de rayons X (2) et le dispositif de positionnement de film (4), grâce à quoi la vitesse de déplacement de la source de rayons X (2) et du dispositif de positionnement de film (4) est commandée au moyen d'une génératrice tachymétrique (26) qui commande la vitesse du servomoteur (16).

2. L'appareil de radiographie à rayons X selon la revendication 1, caractérisé en ce qu'il comprend en outre un intensificateur d'image (5) qui est destiné à recevoir des rayons X qui ont été projetés par la source de rayons X (2) et qui ont traversé la partie désirée du patient (8), à convertir ces rayons en signaux optiques et à visualiser sur un écran d'une caméra de télévision, sous la dépendance des signaux optiques, l'image de la partie qui a été traversée par les rayons X.

3. L'appareil de radiographie à rayons X selon la revendication 1 ou 2, caractérisé en ce que le film est divisé en plusieurs sections et le dispositif de positionnement de film (4) place l'une de ces sections du film à une position à laquelle les rayons X sont projetés à travers l'une des parties désirées du patient, avant que la source de rayons X (2) et le dispositif de positionnement de film (4) ne soient déplacés jusqu'à l'une de leurs positions d'arrêt et que les rayons X ne soient projetés sur la partie désirée du patient (8).

4. L'appareil de radiographie à rayons X selon la revendication 1, 2 ou 3, caractérisé en ce que les moyens de commande (20) comprennent une unité d'asservissement (25) qui est destinée à appliquer des signaux de commande au servomoteur (16), et un moyen pour introduire dans l'unité d'asservissement (25) une information de distance et de vitesse de déplacement de la source de rayons X (2) et du dispositif de positionnement de film (4), lorsque la source de rayons X (2) et le dispositif de positionnement de film (4) doivent être déplacés de l'une de leurs positions d'arrêt vers leur position d'arrêt suivante.

5. L'appareil de radiographie à rayons X selon l'une des revendications 1 à 4, caractérisé en ce que la vitesse de déplacement de la source de rayons X (2) et du dispositif de positionnement de film (4) correspond à la vitesse d'un agent de formation d'image qui circule dans des vaisseaux sanguins dans les parties désirées du patient (8).

6. L'appareil de radiographie à rayons X selon la revendication 4, caractérisé en ce que les moyens de commande (20) comprennent des moyens pour appliquer des signaux à l'unité d'asservissement (25) pour qu'elle commence son action de commande; des moyens pour détecter la position de la source de rayons X (2) et du dispositif de positionnement de film (4), qui sont arrêtés avant que l'unité d'asservissement (25) ne commence son action de commande; et des moyens pour détecter la différence entre la position initiale de la source de rayons X (2) et du dispositif de positionnement de film (4), à laquelle ils commencent à être commandés par l'unité d'asservissement (25), et leur position d'arrêt présente, sous la dépendance de l'information concernant leur position d'arrêt présente, pour fournir l'information concernant cette différence à l'unité d'asservissement (25), afin de les déplacer jusqu'à leur position initiale.

7. L'appareil de radiographie à rayons X selon la revendication 6, caractérisé en ce que les moyens de commande (20) commandent la projection de rayons X sur l'une des parties désirées du patient (8), pour photographier la partie désirée du patient (8) sur l'une des différentes sections du film, avant qu'ils ne commencent à déplacer la source de rayons X (2) et le dispositif de positionnement de film (4) vers leur première position d'arrêt.

8. Un procédé pour commander un appareil de radiographie à rayons X selon la revendication 1, ce procédé comprenant les étapes suivantes :
on déplace la source de rayons X (2) et le dispositif de positionnement de film (4) jusqu'à l'une de leurs positions d'arrêt;
on projette les rayons X sur l'une des parties désirées du patient (8), pour photographier cette partie sur le film, tout en maintenant la source de rayons X (2) et le dispositif de positionnement de film (4) arrêtés à cette position d'arrêt pendant une durée prédéterminée; et
on déplace la source de rayons X (2) et le dispositif de positionnement de film (4) vers leur position d'arrêt suivante, caractérisé en ce qu'on commande la vitesse de déplacement de la source de rayons X (2) et du dispositif de positionnement de film (4) au moyen d'une génératrice tachymétrique (26) qui commande la vitesse du servomoteur (16).
